(19) **Europäisches Patentamt European Patent Office Office européen des brevets**

(11) **EP 1 519 746 B1**

(12) **EUROPEAN PATENT SPECIFICATION**

(45) Date of publication and mention of the grant of the patent:
**15.07.2009 Bulletin 2009/29**

(51) Int Cl.:
*A61K 39/395* (2006.01)  *A61P 9/12* (2006.01)

(21) Application number: **03771818.6**

(22) Date of filing: **24.07.2003**

(86) International application number:
**PCT/US2003/023235**

(87) International publication number:
**WO 2004/011028 (05.02.2004 Gazette 2004/06)**

(54) **USE OF DIGOXIN IMMUNE FAB FOR THE REGULATION OF SODIUM/POTASSIUM ATPASE ACTIVITY IN PREECLAMPTIC AND ECLAMPTIC PATIENTS**

VERWENDUNG VON DIGOXIN-IMMUN-FAB ZUR REGULIERUNG DER NATRIUM/KALIUM-ATPASE-AKTIVITÄT BEI PATIENTEN MIT PRÄEKLAMPSIE UND EKLAMPSIE

UTILISATION D'UN FRAGMENT FAB IMMUNE DE DIGOXINE DANS LA REGULATION DE L'ACTIVITE ATPASE SODIUM/POTASSIUM CHEZ DES PATIENTS PREECLAMPTIQUES ET ECLAMPTIQUES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **25.07.2002 US 202957**
**12.11.2002 US 292338**

(43) Date of publication of application:
**06.04.2005 Bulletin 2005/14**

(73) Proprietor: **Adair, Charles David**
**Signal Mountain, TN 37377 (US)**

(72) Inventor: **Adair, Charles David**
**Signal Mountain, TN 37377 (US)**

(74) Representative: **Hiebl, Inge Elisabeth et al**
**Kraus & Weisert**
**Patent- und Rechtsanwälte**
**Thomas-Wimmer-Ring 15**
**80539 München (DE)**

(56) References cited:
• **ADAIR C D ET AL: "Elevated endoxin-like factor complicating a multifetal second trimester pregnancy: treatment with digoxin-binding immunoglobulin." AMERICAN JOURNAL OF NEPHROLOGY. SWITZERLAND 1996, vol. 16, no. 6, 1996, pages 529-531, XP008025479 ISSN: 0250-8095**

• **GOODLIN R C: "Antidigoxin antibodies in eclampsia." THE NEW ENGLAND JOURNAL OF MEDICINE. UNITED STATES 25 FEB 1988, vol. 318, no. 8, 25 February 1988 (1988-02-25), pages 518-519, XP008025492 ISSN: 0028-4793**

• **CROSSEY MICHAEL J ET AL: "Effects of digoxin-like immunoreactive substances and digoxin FAB antibodies on the new digoxin microparticle enzyme immunoassay" THERAPEUTIC DRUG MONITORING, vol. 19, no. 2, 1997, pages 185-190, XP008025289 ISSN: 0163-4356**

• **KREP HENNING ET AL: "Volume sensitive hypertension and the digoxin-like factor: Reversal by a fab directed against digoxin in DOCA-salt hypertensive rats" AMERICAN JOURNAL OF HYPERTENSION, vol. 8, no. 9, 1995, pages 921-927, XP001176626 ISSN: 0895-7061**

• **GHIONE S ET AL: "Endogenous digitalis-like activity in the newborn" JOURNAL OF CARDIOVASCULAR PHARMACOLOGY, vol. 22, no. SUPPL. 2, 1993, pages S25-S28, XP008025291 ISSN: 0160-2446**

• **GOTO A ET AL: "Ouabain-like factor." CURRENT OPINION IN NEPHROLOGY AND HYPERTENSION. UNITED STATES MAR 1998, vol. 7, no. 2, March 1998 (1998-03), pages 189-196, XP000917769 ISSN: 1062-4821**

• **BAILEY BENOIT: "Are there teratogenic risks associated with antidotes used in the acute management of poisoned pregnant women?" BIRTH DEFECTS RESEARCH, vol. 67, no. 2, February 2003 (2003-02), pages 133-140, XP008025290 ISSN: 1542-0752 (ISSN print)**

Remarks:
The file contains technical information submitted after the application was filed and not included in this specification

Remarks:
The file contains technical information submitted after the application was filed and not included in this specification

## Description

TECHNICAL FIELD OF THE INVENTION

The present invention relates generally to the field of medicine and, more particularly, to a use in a method of regulating sodium/potassium ATPase activity in obstetric patients exhibiting symptoms of preeclampsia and eclampsia by the administration of digoxin immune Fab (ovine).

DESCRIPTION OF THE RELATED ART

Preeclampsia is a rapidly progressive condition occurring during pregnancy characterized by high blood pressure, swelling and protein in the urine. It is specifically defined as the presence of hypertension or pregnancy-induced hypertension ("PIH") accompanied by proteinuria, edema, or both after 20 weeks gestation. Preeclampsia occurs in 5 to 10 percent of all pregnancies and is most common in first-time pregnancies or in first pregnancies with a new partner or husband. Typically, preeclampsia occurs in the late second or third trimesters of pregnancy. Complications of preeclampsia include eclamptic seizures, hemolysis, elevated liver function tests, low platelet count (HELLP) syndrome, hepatic rupture, DIC pulmonary edema, acute renal failure, placental abruption, intrauterine fetal demise (IUFD), cerebral hemorrhage, cortical blindness, and retinal detachment.

Preeclampsia causes vasospasm, which constricts and damages the smooth lining of the blood vessels. This leads to the accumulation of platelets in the damaged blood vessels, which form small clots along the blood vessel wall and further narrow the blood vessel. This damage to blood vessels can also lead to edema, including cerebral edema. Vasospasm can occur throughout the body, damaging the heart, kidneys and liver. Vasospasm can also develop in the placenta, decreasing the blood supply to the fetus and/or placenta.

Preeclampsia is divided into mild and severe forms. Mild preeclampsia is indicated where the patient exhibits hypertension, a proteinuria level of greater than 300 mg per 24 hour period, mild edema signaled by weight gain of greater than 2 pounds per week or 6 pounds per month, and urine output of less than 500 ml per 24 hour period. Severe preeclampsia is indicated where the patient's blood pressure is greater than 160/110 on two occasions at least six hours apart while on bed rest or a systolic blood pressure increase of greater than 60 over a baseline value or a diastolic increase of greater than 30. In addition, a proteinuria level of greater than 5 g per 24 hour period or a reading of 31 or 41 on a urine dipstick, massive edema, oliguria (less than 400 ml per 24 hour period), presence of fetal growth retardation (IUGR), or systemic symptoms including pulmonary edema, headaches, visual changes, right upper quadrant pain, elevated liver enzymes or thrombocytopenia.

After a diagnosis of preeclampsia, the baby is generally induced and delivered if it is near term, *i.e.*, after 36 weeks. However, if preeclampsia occurs earlier in the pregnancy, its impact is more profound. The only "cure" for the disease is delivery of the baby, which is generally contrary to the best interests of the baby if it is not near term. However, if the condition does not respond to traditional management options, early delivery may be the only option remaining. Traditional management includes bed rest, antihypertensive therapy, including methyldopa (Aldomet®), atenolol, and labetalol. If pregnancy from the diagnosis of preeclampsia to delivery could be extended relatively safely for both the fetus and mother, then significant improvement in perinatal outcomes may be achieved.

While the cause of preeclampsia and eclampsia remains unknown, several theories have been propounded. One theory proposes that elevated serum digoxin-like immune factors, a sodium/potassium ATPase enzyme inhibitor, play a central role in the increased peripheral vasoconstriction. This is thought to be mediated through ion exchange pumps resulting in increased intracellular calcium which promotes vasoconstriction and resultant hypertension. Based on this theory, it has been suggested that the use of digoxin immune Fab (ovine) may be productive in controlling preeclampsia and eclampsia and extending pregnancy.

Digoxin immune Fab (ovine), which is marketed in the United States as DIGIBIND® by GlaxoSmithKline and DIGIFAB™ by Protherics, Inc., is a sterile lyophilized powder of antigen binding fragments (FAB) derived from specific antidigoxin antibodies raised in sheep. Digoxin immune Fab (ovine) is indicated for treatment of digoxin or digitoxin overdose manifested by severe ventricular arrhythmias such as ventricular tachycardia or ventricular fibrillation, or progressive bradyarrhythmias such as severe sinus bradycardia or second or third degree heart block not responsive to atropine. DIGIBIND® is distributed in vials, with each vial containing 38 mg of digoxin-specific Fab fragments plus 75 mg of sorbitol as a stabilizer and 28 mg of sodium chloride and capable of binding approximately 0.5 mg of digoxin. DIGIBIND® is generally administered by intravenous injection after reconstitution with 4 mL/vial of sterile water for injection. DIGIFAB™ is distributed in 40 mg vials and contains no preservatives. DIGIFAB™ is generally administered by intravenous infusion over at least thirty minutes after reconstitution with 4 mL/vial of sterile water for injection.

Adair C. D. et al., "Elevated Endoxin-Like Factor Complicating a Multifetal Second Trimester Pregnancy: Treatment With Digoxin-Binding Immunoglobulin", American Journal of Nephrology 1996; 16:529-531 discloses a case report of a second trimester multifetal pregnancy with preeclampsia associated with an elevated digoxin-like immune factor.

The New England Journal of Medicine, Feb. 25, 1988, pages 518-519 discloses the administration of Digibind to a patient experiencing gestational toxemia. Digibind was given to decline the elevated blood pressure which had been non-responsive to a previous antihypertensive therapy but pregnancy had to be terminated and the baby as delivered died immediately post partum.

Crossey M. J. and Dasgupta A., "Effects of Digoxinlike Immunoreactive Substances and Digoxin FAB Antibodies on the New Digoxin Microparticle Enzyme Immunoassay", Department of Pathology, University of New Mexico Health Sciences Center, Albuquerque, New Mexico, USA discloses effects of digoxin-like immunoreactive substances and digoxin FAB antibodies on the new digoxin microparticle enzyme immunoassay.

The present invention is directed to overcoming one or more of the problems set forth above.

SUMMARY OF THE INVENTION

The present invention relates to the use of antigen-binding Fab fragments, called "digoxin immune Fab (ovine)", derived from specific antidigoxin antibodies raised in sheep, for the preparation of a medicament for the treatment of preeclampsia / eclampsia, wherein the treatment comprises administering a suitable dosage of digoxin immune Fab (ovine) according to a formula of an endogenous digitalis-like factor level of between approximately 3.0 and 5.0 ng/mL times the patient's body weight in kilograms divided by 100 via an intravenous bolus and repeating the administration of a suitable dosage of digoxin immune Fab (ovine) on a fixed schedule.

The present invention further relates to antigen-binding Fab fragments, called "digoxin immune Fab (ovine)", derived from specific antidigoxin antibodies raised in sheep, for use in a method of treatment of preeclampsia / eclampsia, wherein the treatment comprises administering a suitable dosage of digoxin immune Fab (ovine) according to a formula of an endogenous digitalis-like factor level of between approximately 3.0 and 5.0 ng/mL times the patient's body weight in kilograms divided by 100 via an intravenous bolus and repeating the administration of a suitable dosage of digoxin immune Fab (ovine) on a fixed schedule.

The present invention further relates to antigen-binding Fab fragments, called "digoxinimmune Fab (ovine)", derived from specific anti-digoxin antibodies raised in sheep, for use as detailed above, wherein the treatment schedule further includes the step of administering a suitable dosage of corticosteroids, and whereby pregnancy is extended.

BRIEF DESCRIPTION OF THE DRAWINGS

Reference is now made more particularly to the drawings which illustrate the best presently known mode of carrying out the invention and wherein similar reference characters indicate the same parts throughout the views.

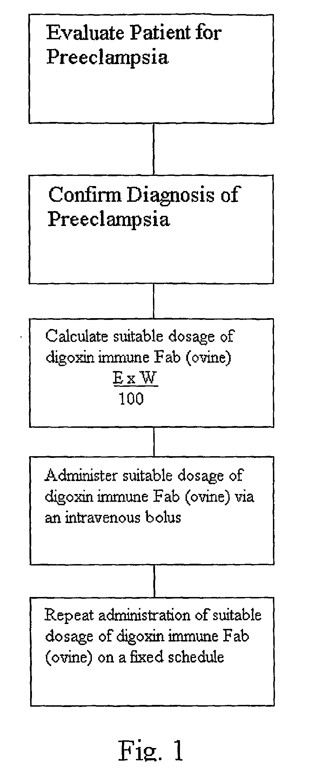

Fig. 1 is a flow chart illustrating the steps and sequence of a method for controlling preeclampsia according to the use of the present invention.

Fig. 2A is a chart of the blood flow velocity through a patient's umbilical artery in a normal pregnancy.

Fig. 2B is a chart of the blood flow velocity through a pre-eclamptic patient's umbilical artery.

DETAILED DESCRIPTION

Patients suffering from preeclampsia and eclampsia experience vascular contraction, retention of cellular sodium (Na), and expansion of intracellular volume. It is theorized that these conditions in preeclamptic and eclamptic patients are related to the occurrence of faulty invasion of the placenta into the uterine wall and a corresponding reaction by the fetus and/or placenta to that faulty invasion. In the early stages of pregnancy, the placenta has a "loose" connection with the uterine wall. Around the fourteenth to sixteenth week of pregnancy, the placenta develops a "tight" attachment to the uterine wall in order to tap more fully into the maternal blood supply. This tighter connection between the placenta and uterine wall allows for the exchange of larger and more complex nutrients that are required for the continued development of the fetus at that stage of the pregnancy. In certain instances, the placenta is unable to fully develop the necessary tight connection with the uterine wall. This faulty connection makes the exchange of the required nutrients between the mother and fetus and/or placenta more difficult and places stress on the fetus. The reaction of the fetus to this stress is the generation of a compensatory mechanism. As part of this compensatory mechanism, the fetus and/or placenta produces and transfers to the mother an endogenous digitalis-like factor(s).

It is further theorized that the endogenous digitalis-like factor(s) binds to the mother's sodium/potassium ATPase in a manner similar to digoxin or digitoxin. Digoxin is a toxin that binds to and inhibits the activity of sodium/potassium ATPase pumps in cells of the human body. The resulting inhibition of these ATPase pumps produces an imbalance of sodium and potassium in the intra and extra cellular space. In cases of severe digitalis intoxication, a patient's serum potassium concentration can be elevated to life-threatening levels, due to the shifting of potassium from inside to outside of the patient's cells. This leads to increased renal excretion of potassium and, possibly, to hyperkalemia with a total body deficit of potassium. Other symptoms of severe digitalis intoxication include severe ventricular arrhythmias, such as ventricular tachycardia or ventricular fibrillation, or progressive bradyarrhythmias, such as severe sinus bradycardia or second or third degree heart block not responsive to atropine.

According to this theory, the endogenous digitalis-like factor(s) produced by the fetus and/or placenta creates a similar effect on the cells of a preeclamptic patient. The endogenous digitalis-like factor(s) binds to and inhibits the activity of the patient's sodium/potassium ATPase. The inhibition of sodium/potassium ATPase results in elevated intracellular sodium and, consequently, elevated intracellular calcium. The elevated levels of intracellular sodium lead to intravascular volume contraction and vasoconstriction in the patient. The vasoconstriction experienced by the patient can produce exaggerated myocardial function due to the increased effort required by the heart to pump blood through the patient's narrowed vascular system. It is also possible that this exaggerated myocardial function is produced by the endogenous digitalis-like factor(s) binding to cells in the patient's heart.

Due to the similarity of effects of digoxin and the endogenous digitalis-like factor(s), it is theorized that the drug used for treatment of digitalis intoxication may prove effective for the treatment of preeclampsia and eclampsia. Digitalis intoxication is treated through the administration of digoxin immune Fab (ovine). Digoxin immune Fab (ovine), which is marketed in the United States as DIGIBIND® by GlaxoSmithKline and DIGIFAB™ by Protherics, Inc., is a sterile lyophilized powder of antigen binding fragments (FAB) derived from specific antidigoxin antibodies raised in sheep. Digoxin immune Fab (ovine) is indicated for treatment of digoxin or digitoxin overdose manifested by severe ventricular arrhythmias such as ventricular tachycardia or ventricular fibrillation, or progressive bradyarrhythmias such as severe sinus bradycardia or second or third degree heart block not responsive to atropine. DIGIBIND® is distributed in vials, with each vial containing 38 mg of digoxin-specific Fab fragments plus 75 mg of sorbitol as a stabilizer and 28 mg of sodium chloride and capable of binding approximately 0.5 mg of digoxin. DIGIBIND® is generally administered by intravenous injection after reconstitution with 4 mL/vial of sterile water for injection. DIGIFAB™ is distributed in 40 mg vials and contains no preservatives. DIGIFAB™ is generally administered by intravenous infusion over at least thirty minutes after reconstitution with 4 mL/vial of sterile water for injection. Digoxin immune Fab (ovine) binds to molecules of digoxin, thereby preventing them from binding to and inhibiting the activity of the body's sodium/potassium ATPase pumps. This is due to the fact that the affinity of digoxin immune Fab (ovine) is significantly greater than the affinity for sodium/potassium ATPase. The Fab fragment-digoxin combined molecule then accumulates in the blood and is excreted by the kidneys.

It is theorized that administration of digoxin immune Fab (ovine) to preeclamptic and eclamptic patients will result in the digoxin immune Fab (ovine) binding to the endogenous digitalis-like factor(s), thereby preventing the endogenous digitalis-like factor(s) from binding to and inhibiting the activity of the patient's sodium/potassium ATPase pumps and leading to the eventual excretion of the endogenous digitalis-like factor(s) through the kidneys. This, in turn, should allow the patient's levels of intracellular sodium and calcium to normalize and ease any intravascular volume contraction and vasoconstriction, further leading to normal myocardial function.

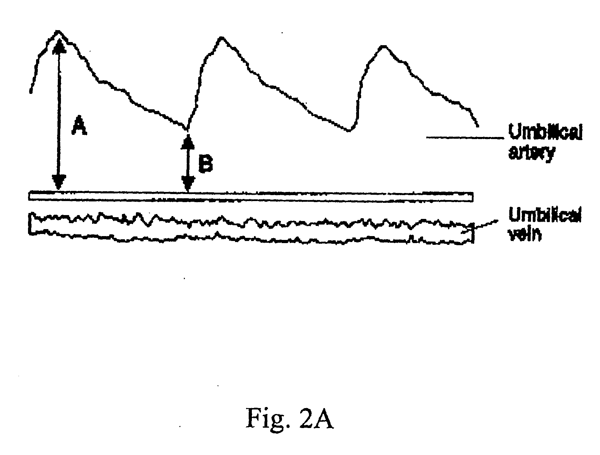

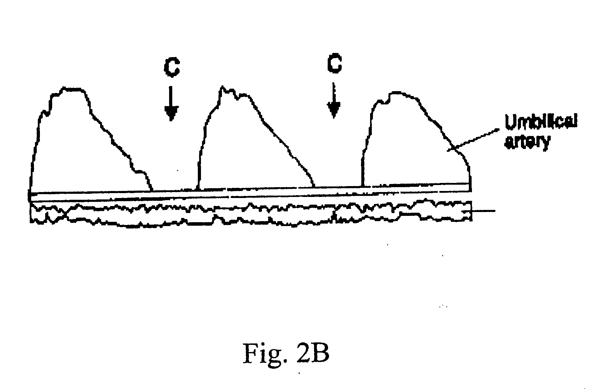

The vasoconstriction and exaggerated myocardial function experienced by pre-eclamptic and eclamptic patients also negatively impacts the flow of blood to the fetus and/or placenta. Fig. 2A illustrates the blood flow velocity in the umbilical artery of a normal pregnancy. "A" refers to the blood flow velocity during systole, while "B" refers to the blood flow velocity in the umbilical artery during diastole. Fig. 2A illustrates that even during diastole, there is a significant flow velocity through the umbilical artery. This is in contrast to the flow velocity in a pre-eclamptic patient as shown in Fig. 2B. Pre-eclamptic vasoconstriction reduces the blood flow velocity through the umbilical artery and results in the flow velocity "bottoming out" during diastole, indicated by "C". During diastole, little or no blood is flowing to the fetus and/or placenta, placing further stress on the fetus and restricting oxygenation, fluid exchange and nutrition, potentially resulting in growth restriction of the fetus. It is theorized that administration of digoxin immune (Fab) ovine to an obstetric patient suffering from preeclampsia or eclampsia eases vasoconstriction, thereby allowing the fetal blood flow to improve.

According to one example, a patient is first evaluated for preeclampsia or eclampsia. Once a diagnosis of preeclampsia is confirmed, a suitable dosage of digoxin immune Fab (ovine), such as DIGIBIND® or DIGIFAB™, is calculated. A typical manufacturer's recommended dosage formula for a known digitalis toxicity level (E) is:

$$\frac{D \times W}{100}$$

where (D) is the serum digoxin concentration in ng/mL and W is the patient's weight in kilograms. However, in preeclampsia and eclampsia cases there is no known digitalis toxicity level. Therefore, according a preferred method, a measured or assumed endogenous digitalis factor(s) level is utilized for the calculation. According to the invention, the endogenous digitalis-like factor(s) level (E) is between approximately 3.0 and 5.0 ng/mL. In a preferred embodiment, the endogenous digitalis-like factor(s) level is approximately 4.0 ng/mL. The endogenous digitalis-like factor(s) level is then used in the following equation:

$$\frac{E \times W}{100}$$

where E is the endogenous digitalis-like factor(s) level. Use of the preferred endogenous digitalis-like factor(s) level results in an equation of:

$$\frac{4.0\ ng/mL \times (W)}{100}$$

which in turn produces the suitable dosage of digoxin immune Fab (ovine).

Once the dosage is calculated, it is administered to the patient via an intravenous bolus. Administration of the calculated dosage via intravenous bolus is then repeated on a fixed schedule of every five to eight hours, preferably every six hours.

In another embodiment, the administration of digoxin immune Fab (ovine) is accompanied by an administration of corticosteroids, such as betamethasone, to stimulate the cells in the lungs of the fetus in preparation for breathing air and decrease the risk of brain hemorrhage and intestinal problems in the fetus. The corticosteroids are administered once at the time of admission and again in twenty-four hours.

The method will now be further illustrated with reference to the following non-limiting example.

Biological Example

A 16-year-old 70 Kg primigravida at 29 weeks 5/7 days presented with a presumed diagnosis of eclampsia. Her prenatal course had been uncomplicated until the day of admission. Her past medical history was negative for chronic illness. The patient had complaints of scotomata, persistent headache, and reported seizure activity for two episodes prior to hospital arrival and one on the labor deck. Initial evaluation revealed an alert patient with obvious postictal behavior. Her blood pressure was elevated at 160/110's and otherwise stable vital signs. Physical examination revealed A-V nicking on funduscopic exam, 4+ edema of the lower extremities and obvious facial and upper extremity edema. Deep tendon reflexes were 3 plus with 2 beats of clonus. The remainder of the exam was within normal limits. Lab evaluation of the urine noted proteinuria on qualitative analysis of 2+ and a urine specific gravity of 1.025. Serum chemistry revealed hyperuricacidemia at 8.2, elevated creatinine of 1.0, BUN of 6, and otherwise normal electrolytes and liver enzymes. The complete blood count revealed a platelet count of 429,000, white count of 10.4 and a hemoglobin/hematocrit of 12.0 and 35.6 respectively. The ammonia level was 3 and coagulation studies were normal. Urine drug screening was negative for substances of abuse including cocaine and methamphetamines. CT scanning of the maternal head failed to reveal any pathological abnormalities.

Ultrasound examination revealed a singleton gestation with a breech presentation. The estimated gestational age was consistent with the previously determined age of 29 weeks with an estimated fetal weight of 1331 grams. The amniotic fluid index was 5.42 cm and fetal breathing, movement, and tone were noted to be present. Doppler flow studies of the umbilical artery revealed an elevated S/D ratio of 5.6, RI of .82, and minimal diastolic flow. No anatomical abnormalities were noted on exam. Fetal cardiotocograph revealed a baseline of 135 with minimal beat-to-beat variability. Occasional non-repetitive decelerations were noted with good recovery. They were deemed to be non-ominous.

The patient was placed on intravenous magnesium sulfate. Central venous access and arterial line placement were performed. After informed discussion with the immediate family including the option to stabilize with compassionate off label use of fragmented antibody to digoxin and to administer corticosteroids for fetal benefit, the patient's mother provided consent.

Standard preeclampsia monitoring every hour was followed with one on one nursing in the intensive care unit. Using an endogenous digitalis like factor(s) level of 4.0 ng/mL and a dosage formula of 4.0 ng/mL x patient's body weight divided by 100 resulted in a dosage of 3 vials. This dosage was administered via an intravenous bolus and was repeated every 6 hours.

Intravenously administered fluids were standardized to 125 cc/hr. Urine output from admission to time of digoxin immune Fab (ovine) infusion was 300 cc. Over the first 6 hours post infusion of digoxin immune Fab (ovine), the average urinary output increased to 100 cc/hr with blood pressure readings of 148 to 162 systolic and 104 to 111 diastolic. At the next dosing interval the drug was doubled to 6 vials every six hours until 12 hours prior to delivery. Over the next 36 hours a diuresis followed over 5,840 cc of urine for an average hourly output of 162 cc. The total intake of intravenous fluids during the same time period was 3409 cc. The urine qualitative exam revealed negative proteinuria with a specific gravity of 1.011 and osmolality of 125, normal 500 to 800 mosm. Her creatinine decreased to 0.7 with the remainder of electrolytes remaining normal. Serial blood pressure measurements during the 36-hour period from the doubling of the dose ranged from 131 to 160 systolic (mean 140's) and diastolic 58 to 111 (mean 90's). The edema resolved in face and upper extremities with significant decreases in lower extremities as well to 1+. Deep tendon reflexes became 1+ with no clonus.

Fetal assessment during this time showed no changes in the cardiotocograph. Ultrasound examination every 6 hours revealed a reassuring biophysical score of 8 of 10. Doppler flow assessment of the umbilical artery every 6 hours revealed a decreasing systolic/diastolic ratio with an increasing diastolic component. The S/D ratio 6 hours prior to delivery was 3.7 with a RI of .75 suggesting improved fetal hemodynamics albeit still abnormally elevated.

Cesarean delivery was performed at 48 hours post administration of corticosteroids secondary to a breech presentation. The delivery was attended by NICU and resulted in a livebirth of a female child. Apgars were 7 & 8 at one and five minutes respectively. The neonate did not require any oxygen support and was admitted to the neonatal intensive care unit secondary to prematurity size of 1290 grams.

The maternal postoperative course was complicated by elevated blood pressures of 140 to 160's systolic and diastolic readings in the 100's. The patient was started on metoprolol 100 mg twice daily. She was discharged home on postoperative day # 4 with stable blood pressures controlled with metoprolol. All follow up laboratory tests remained within normal limits. The neonate had no adverse sequelae and was discharged home on day of life # 31.

**Claims**

1. The use of antigen-binding Fab fragments, called "digoxin immune Fab (ovine)", derived from specific antidigoxin antibodies raised in sheep, for the preparation of a medicament for the treatment of preeclampsia / eclampsia, wherein the treatment comprises administering a suitable dosage of digoxin immune Fab (ovine) according to a formula of an endogenous digitalis-like factor level of between approximately 3.0 and 5.0 ng/mL times the patient's body weight in kilograms divided by 100 via an intravenous bolus and repeating the administration of the suitable dosage of digoxin immune Fab (ovine) on a fixed schedule.

2. The use of a digoxin immune Fab (ovine) as set forth in claim 1, wherein the endogenous digitalis-like factor level is approximately 4.0 ng/mL.

3. The use of a digoxin immune Fab (ovine) as set forth in claim 1 or 2, wherein the fixed schedule is approximately every five to eight hours.

4. The use of a digoxin immune Fab (ovine) as set forth in claim 3, wherein the fixed schedule is approximately every six hours.

5. The use of a digoxin immune Fab (ovine) as set forth in one of claims 1 to 4, wherein the initial dosage of digoxin immune Fab (ovine) is doubled at the repeated administration.

6. The use of one of claims 1 to 5, wherein the treatment schedule further includes the step of administering a suitable dosage of corticosteroids, and whereby pregnancy is extended.

7. The use of one of claims 2 to 6, wherein the treatment schedule further includes the step of administering intravenous fluids,

8. Antigen-binding Fab fragments, called "digoxin immune Fab (ovine)", derived from specific antidigoxin antibodies raised in sheep, for use in a method of treatment of preeclampsia / eclampsia, wherein the treatment comprises administering a suitable dosage of digoxin immune Fab (ovine) according to a formula of an endogenous digitalis-like factor level of between approximately 3.0 and 5.0 ng/mL times the patient's body weight in kilograms divided by 100 via an intravenous bolus and repeating the administration of the suitable dosage of digoxin immune Fab (ovine) on a fixed schedule.

9. The digoxin immune Fab (ovine) of claim 8, wherein the endogenous digitalis-like factor level is approximately 4.0 ng/mL.

10. The digoxin immune Fab (ovine) of claim 8 or 9, wherein the fixed schedule is approximately every five to eight hours.

11. The digoxin immune Fab (ovine) of claim 10, wherein the fixed schedule is approximately every six hours.

12. The digoxin immune Fab (ovine) of claims 8 to 11, wherein the initial dosage of digoxin immune Fab (ovine) is doubled at the repeated administration.

13. Antigen-binding Fab fragments, called "digoxin immune Fab (ovine)", derived from specific antidigoxin antibodies raised in sheep, for use according to one of claims 8 to 12, wherein the treatment schedule further includes the step of administering a suitable dosage of corticosteroids, and whereby pregnancy is extended.

14. The digoxin immune Fab (ovine) of one of claims 9 to 13, wherein the treatment schedule further includes the step of administering intravenous fluids.

**Patentansprüche**

1. Verwendung von Antigen-bindenden Fab-Fragmenten, "(Schaf-) Digoxin-Immun-Fab" genannt, abgeleitet von spezifischen Anti-Digoxin-Antikörpern, die in Schafen entwickelt wurden, für die Herstellung eines Medikaments zur Behandlung von Präeklampsie/Eklampsie, wobei die Behandlung Verabreichen einer geeigneten Dosierung von (Schaf-) Digoxin-Immun-Fab entsprechend einer Formel: Konzentration von endogenem Digitalis-ähnlichem Faktor von zwischen etwa 3,0 und 5,0 ng/ml mal das Körpergewicht des Patienten in Kilogramm, dividiert durch 100, über einen intravenösen Bolus und Wiederholen der Verabreichung der geeigneten Dosierung von (Schaf-) Digoxin-Immun-Fab nach einem festgelegten Plan umfasst.

2. Verwendung eines (Schaf-) Digoxin-Immun-Fab, wie in Anspruch 1 angegeben, wobei die Konzentration von endogenem Digitalis-ähnlichem Faktor etwa 4,0 ng/ml ist.

3. Verwendung eines (Schaf-) Digoxin-Immun-Fab, wie in Anspruch 1 oder 2 angegeben, wobei der festgelegte Plan etwa alle fünf bis acht Stunden ist.

4. Verwendung eines (Schaf-) Digoxin-Immun-Fab, wie in Anspruch 3 angegeben, wobei der festgelegte Plan etwa alle sechs Stunden ist.

5. Verwendung eines (Schaf-) Digoxin-Immun-Fab, wie in einem der Ansprüche 1 bis 4 angegeben, wobei die Anfangsdosierung von (Schaf-) Digoxin-Immun-Fab bei der wiederholten Verabreichung verdoppelt wird.

6. Verwendung gemäß einem der Ansprüche 1 bis 5, wobei der Behandlungsplan außerdem den Schritt eines Verabreichens einer geeigneten Dosierung von Corticosteroiden umfasst und wodurch die Schwangerschaft verlängert wird.

7. Verwendung gemäß einem der Ansprüche 2 bis 6, wobei der Behandlungsplan außerdem den Schritt eines Verabreichens von intravenösen Flüssigkeiten umfasst.

8. Antigen-bindende Fab-Fragmente, "(Schaf-) Digoxin-Immun-Fab" genannt, abgeleitet von spezifischen Anti-Digoxin-Antikörpern, die in Schafen entwickelt wurden, zur Verwendung in einem Verfahren zur Behandlung von Präeklampsie/Eklampsie, wobei die Behandlung Verabreichen einer geeigneten Dosierung von (Schaf-) Digoxin-Immun-Fab entsprechend einer Formel: Konzentration von endogenem Digitalis-ähnlichem Faktor von zwischen etwa 3,0 und 5,0 ng/ml mal das Körpergewicht des Patienten in Kilogramm, dividiert durch 100, über einen intravenösen Bolus und Wiederholen der Verabreichung der geeigneten Dosierung von (Schaf-) Digoxin-Immun-Fab nach einem festgelegten Plan umfasst.

9. (Schaf-) Digoxin-Immun-Fab gemäß Anspruch 8, wobei die Konzentration von endogenem Digitalis-ähnlichem Faktor etwa 4,0 ng/ml ist.

10. (Schaf-) Digoxin-Immun-Fab gemäß Anspruch 8 oder 9, wobei der festgelegte Plan etwa alle fünf bis acht Stunden ist.

11. (Schaf-) Digoxin-Immun-Fab gemäß Anspruch 10, wobei der festgelegte Plan etwa alle sechs Stunden ist.

12. (Schaf-) Digoxin-Immun-Fab gemäß den Ansprüchen 8 bis 11, wobei die Anfangsdosierung von (Schaf-) Digoxin-Immun-Fab bei der wiederholten Verabreichung verdoppelt wird.

13. Antigen-bindende Fab-Fragmente, "(Schaf-) Digoxin-Immun-Fab" genannt, abgeleitet von spezifischen Anti-Digoxin-Antikörpern, die in Schafen entwickelt wurden, zur Verwendung gemäß einem der Ansprüche 8 bis 12, wobei der Behandlungsplan außerdem den Schritt eines Verabreichens einer geeigneten Dosierung von Corticosteroiden umfasst und wodurch eine Schwangerschaft verlängert wird.

14. (Schaf-) Digoxin-Immun-Fab gemäß einem der Ansprüche 9 bis 13, wobei der Behandlungsplan außerdem den Schritt eines Verabreichens von intravenösen Flüssigkeiten umfasst.

**Revendications**

1. Utilisation de fragments Fab liant antigène, dits «digoxin immune Fab (ovine)» dérivés d'anticorps anti-digoxine spécifiques, développés dans le mouton, pour la fabrication d'un médicament destiné au traitement de la prééclampsie/éclampsie, le traitement comprenant l'administration d'un dosage approprié de «digoxin immune Fab (ovine)» satisfant la formule: la concentration de facteur similaire à digitale endogène entre environ 3,0 et 5,0 ng/ml fois le poids du corps du patient en kg divisé par 100, via un bolus intraveineux et la répétition de l'administration du dosage approprié de «digoxin immune Fab (ovine)» selon un plan fixe.

2. Utilisation d'un «digoxin immune Fab (ovine)» selon la revendication 1, dans laquelle la concentration de facteur similaire à digitale endogène est environ 4,0 ng/ml.

3. Utilisation d'un «digoxin immune Fab (ovine)» selon la revendication 1 ou 2, dans laquelle le plan fixe comprend une administration environ toutes les cinq à huit heures.

4. Utilisation d'un «digoxin immune Fab (ovine)» selon la revendication 3, dans laquelle le plan fixe comprend une administration environ tous les six heures.

5. Utilisation d'un «digoxin immune Fab (ovine)» selon l'une quelconque des revendications 1 à 4, dans laquelle le dosage de «digoxin immune Fab (ovine)» est doublé à l'administration répétée.

6. Utilisation selon l'une quelconque des revendications 1 à 5, dans laquelle le plan d'administration comprend en outre l'étape d'administrer un dosage approprié de corticostéroïdes, et ainsi la grossesse est prolongée.

**7.** Utilisation selon l'une quelconque des revendications 2 à 6, dans laquelle le plan d'administration comprend en outre l'étape d'administrer des liquides intraveineux.

**8.** Fragment Fab liant antigène, dits «digoxin immune Fab (ovine)», dérivés d'anticorps anti-digoxine spécifiques, développés dans le mouton, pour la fabrication d'un médicament destiné au traitement de la prééclampsie/éclampsie,
le traitement comprenant l'administration d'un dosage approprié de «digoxin immune Fab (ovine)» satisfant la formule: la concentration de facteur similaire à digitale endogène entre environ 3,0 et 5,0 ng/ml fois le poids du corps du patient en kg divisé par 100, via un bolus intraveineux et la répétition de l'administration du dosage approprié de «digoxin immune Fab (ovine)» selon un plan fixe.

**9.** «Digoxin immune Fab (ovine)» selon la revendication 8, dans lequel la concentration de facteur similaire à digitale endogène est environ 4,0 ng/ml.

**10.** «Digoxin immune Fab (ovine)» selon la revendication 8 ou 9, dans lequel le plan fixe comprend une administration environ toutes les cinq à huit heures.

**11.** «Digoxin immune Fab (ovine)» selon la revendication 8 ou 9, dans lequel le plan fixe comprend une administration environ tous les six heures.

**12.** «Digoxin immune Fab (ovine)» selon la revendication 8 ou 9, dans lequel le dosage initial de «digoxin immune Fab (ovine)» est doublé à l'administration répétée.

**13.** Fab fragments liant antigène, dit «digoxin immune Fab (ovine)», dérivés d'anticorps anti-digoxine spécifiques, développés dans le mouton, pour l'utilisation selon l'une quelconque des revendications 8 à 12, le plan d'administration comprenant en outre l'étape d'administrer un dosage approprié des corticostéroïdes, et ainsi la grossesse étant prolongée.

**14.** «Digoxin immune Fab (ovine)» selon la revendication 9 à 13, le plan de traitement comprend en outre l'étape d'administrer des liquides intraveineux.

Evaluate Patient for
Preeclampsia

Confirm Diagnosis of
Preeclampsia

Calculate suitable dosage of
digoxin immune Fab (ovine)
$$\frac{E \times W}{100}$$

Administer suitable dosage of
digoxin immune Fab (ovine) via
an intravenous bolus

Repeat administration of suitable
dosage of digoxin immune Fab
(ovine) on a fixed schedule

Fig. 1

Fig. 2A

Fig. 2B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Adair C. D. et al.** Elevated Endoxin-Like Factor Complicating a Multifetal Second Trimester Pregnancy: Treatment With Digoxin-Binding Immunoglobulin. *American Journal of Nephrology,* 1996, vol. 16, 529-531

- *The New England Journal of Medicine,* 25 February 1988, 518-519
- **Crossey M. J. ; Dasgupta A.** *Effects of Digoxinlike Immunoreactive Substances and Digoxin FAB Antibodies on the New Digoxin Microparticle Enzyme Immunoassay*